# EUROPEAN PATENT APPLICATION

(11) **EP 2 767 967 A1**
(43) Date of publication of application: **20.08.2014**
(21) Application number: 12839556.3
(22) Date of filing: 17.08.2012
(51) Int. Cl.: G09B 5/06, G09B 19/00, G06Q 50/20

(54) **APPARATUS FOR TRAINING RECOGNITION CAPABILITY USING ROBOT AND METHOD FOR SAME**

(30) Priority: 12.10.2011 KR 20110104249
(71) Applicant: Korea Institute of Science and Technology, Seoul 136-791 (KR)
(72) Inventor: KIM, Mun Sang, Seoul 135-100 (KR); CHOI, Mun Taek, Uijeongbu-si Gyeonggi-do 480-712 (KR); YUN, Sang Seok, Busan 611-074 (KR); BAEK, Kyoung Keun, Seoul 136-819 (KR); LEE, Seong Whan, Seoul 135-838 (KR)
(74) Representative: advotec.
(86) International application number: PCT/KR2012/006543
(87) International publication number: WO 2013/055024

(57) **Abstract**

Disclosed is an apparatus for training a recognition capability by using a robot and to a method for same. Disclosed is the apparatus for training the recognition capability, according to one embodiment of the present invention, comprising: an instruction generation portion for transmitting to the robot a series of robot instructions for controlling the behavior of the robot; a sensor portion for collecting sensor information including a 3D position information and color information of a trainee; a trainee behavioral information generation portion for generating behavioral information of the trainee, based on the sensor information that is collected; and a recognition capability determination portion for outputting the recognition capability of the trainee, based on the robot instructions and the behavioral information of the trainee.

## Description

### [Technical Field]

Embodiments of the present disclosure relate to an apparatus and method for training recognition ability. More particularly, embodiments of the present disclosure relate to an apparatus for training recognition ability using a robot and a method for evaluating recognition ability.

### [Background Art]

Recognition ability refers to the ability for distinguishing, selecting, accepting, understanding and possessing suitable information, and when the information is required, searching and suitably applying relevant information. If cognitive impartment occurs, the efficiency for information treatment deteriorates, and the speed and durability of the recognition function also deteriorate, which lowers the functions for daily life and makes it difficult to suitably cope with problems. In addition, patients suffering from structural damages of the brain such as traumatic brain injury, cerebral infraction or cerebral palsy are inevitably companied by the disorder of various brain functions, and some of such patients are accompanied by the disorder of exercise functions, the disorder of recognition ability such as attention concentration, memory, judgment, problem-solving ability, planning ability or the like, particularly the disorder of perception ability for accepting and processing information from sensory organs.

The disorder of cognitive function and perception ability result in serious disorders in rehabilitation to the society or normal social life after brain damage. For treating such patients, accurate and comprehensive evaluation should be preferentially performed, and problems found by such an accurate evaluation should be intensively treated. Basically, various medicine treatments, physical treatment, work treatment, psychological treatment, language treatment or the like is performed, and recently various diagnosis and treatment programs using a developed computer technique have been developed and practically used in relation to the disorder of cognitive impartment and perception ability. However, most of the computer recognition treatment programs developed and used until now are not yet actively used clinically.

Korean Patent Application Publication No. 10-2008-0005798 discloses a recognition and behavior disorder rehabilitating system using a motion tracking technique and an augmented reality method. In order to implement a recognition and behavior disorder rehabilitating system which is not boring and also solves inconvenience caused by the manipulation of a keyboard or a mouse, an interested color is extracted and a CAMSHIFT algorithm is applied. The interested color is extracted by conversion into a HSV color space, and only the interested color is used in order to reduce other noise. In addition, a cognitive impartment rehabilitation supporting system for performing a visual reaction function, a visual differential reaction function and a visual reaction and move function for measuring attentiveness and reaction time and a behavior disorder rehabilitation supporting system for performing a visual track and target function to measure attention concentration ability and hand motion accommodation ability and a visual spatial and motor function to measure visual exercise ability are disclosed. However, the rehabilitation supporting system gives just two-dimensional information to a patient by using a display device having a computer monitor, and the recognition ability of the patent could be treated only with two-dimensional information. For this reason, only a simple gesture may be expressed to the patient, which has a limit in recognition treatment. In addition, when a body of the patient is used, even though a simple gesture for hand motion accommodation or the like is possible, an exercise using the whole body of the patient or extended activity may not be easily treated.

### [Disclosure]

### [Technical Problem]

An aspect of the present disclosure is directed to treating recognition ability in a three-dimensional real space by delivering an instruction to a patient by means of a robot.

Another aspect of the present disclosure is directed to treating various kinds of recognition ability utilizing the whole body of a patient.

Another aspect of the present disclosure is directed to improving space perception ability, short term memory ability, eyesight and exercise ability of a patient.

### [Technical Solution]

According to an embodiment, there is provided an apparatus for training recognition ability, which includes: an instruction generating unit for generating a series of robot instruction information to instruct a behavior of a robot or a series of trainee instruction information to instruct a behavior of a trainee; a sensor unit for collecting three-dimensional location information of the trainee who imitates the behavior of the robot according to the robot instruction information or acts according to the trainee instruction information, and sensor information including color information or mobile device usage information of the trainee; a trainee behavior information generating unit for generating trainee behavior information based on the sensor information collected by the sensor unit; and a recognition ability determining unit for calculating recognition ability of the trainee based on the trainee behavior information along with the robot instruction information or the trainee instruction information.

According to another embodiment, the apparatus for training recognition ability may further include: a first communication unit capable of communicating with an arbitrary object external to the apparatus for training recognition ability; a display unit for providing visual information to the trainee; and a voice unit for providing voice information to the trainee, wherein the series of robot instruction information is transmitted to the robot through the first communication unit, and the trainee instruction information is delivered to the trainee through the display unit or the voice unit.

According to another embodiment, the mobile device usage information may be generated from at least one of a touch sensor, an acceleration sensor, a magnetic sensor and a rotation sensor in the mobile device, and when the trainee instruction information is an instruction for operating the robot by using the mobile device, the instruction generating unit may generate a series of robot instruction information again.

According to another embodiment, when it is determined that the robot instruction information or the trainee instruction information is identical to the trainee behavior information within a predetermined range, the instruction generating unit may generate robot instruction information for controlling the behavior of the robot again so that the robot performs a feedback behavior or feedback information is provided to the trainee through the display unit or the voice unit.

According to another embodiment, the voice information may guide a time limit for the trainee to imitate the robot instruction information or act according to the trainee instruction information, the time limit being adjustable.

According to another embodiment, the instruction generating unit may generate the series of robot instruction information or trainee instruction information based on trainee information, and the trainee information may include face color information, gender information and age information of the trainee, and usage information of the apparatus for training recognition ability.

According to another embodiment, the instruction generating unit may include: a movement instruction generating unit for generating movement instruction to change a location of the robot; and a gesture instruction generating unit for generating a gesture instruction to change a motion of the robot.

According to another embodiment, the sensor unit may include: a location sensor unit for collecting three-dimensional location information of the trainee; a red-green-blue (RGB) sensor unit for collecting color information of the trainee; and a mobile device sensor unit for collecting mobile device usage information of the trainee.

According to another embodiment, the apparatus for training recognition ability may further include a trainee information managing unit for determining whether the trainee is already registered by using the collected trainee color information and registering the trainee information.

According to another embodiment, the trainee information managing unit may include: a trainee face searching unit for searching corresponding trainee information from pre-stored trainee information based on face color information among the collected trainee color information; and a trainee information registering unit for registering trainee information including face color information among the collected trainee color information, if a corresponding face is not searched based on the collected trainee face information.

According to another embodiment, the trainee behavior information generating unit may divide an available place of the trainee into a plurality of spaces, and generate the trainee behavior information by matching three-dimensional location information of the trainee with the plurality of spaces.

According to another embodiment, the trainee behavior information generating unit may generate the trainee behavior information by calculating a bending angle of a physical joint of the trainee based on the three-dimensional location information of the trainee.

According to another embodiment, there is provided a method for training recognition ability, which includes: generating a series of robot instruction information to instruct a behavior of a robot or a series of trainee instruction information to instruct a behavior of a trainee; collecting three-dimensional location information of the trainee who imitates the behavior of the robot according to the robot instruction information or acts according to the trainee instruction information, and sensor information including color information or mobile device usage information of the trainee; generating trainee behavior information based on the sensor information collected by the sensor unit; and calculating recognition ability of the trainee based on the trainee behavior information along with the robot instruction information or the trainee instruction information.

According to another embodiment, the generating a series of robot instruction information or a series of trainee instruction information may further include: transmitting the series of robot instruction information to the robot; and delivering the trainee instruction information to the trainee as visual information through a display unit or voice information through a voice unit.

According to another embodiment, the generating a series of robot instruction information or a series of trainee instruction information may further include generating a series of robot instruction information again when the trainee instruction information is an instruction for operating the robot by using the mobile device.

According to another embodiment, in the calculating of recognition ability of the trainee based on the trainee behavior information along with the robot instruction information or the trainee instruction information, when it is determined that the robot instruction information or the trainee instruction information is identical to the trainee behavior information within a predetermined range, robot instruction information for controlling the behavior of the robot may be generated again so that the robot performs a feedback behavior or feedback information is provided to the trainee through the display unit or the voice unit.

According to another embodiment, the voice information may guide a time limit for the trainee to imitate the robot instruction information or act according to the trainee instruction information, the time limit being adjustable.

According to another embodiment, the generating of a series of robot instruction information or a series of trainee instruction information may further include generating the series of robot instruction information or trainee instruction information based on trainee information, and the trainee information may include face color information, gender information and age information of the trainee, and usage information of the apparatus for training recognition ability.

According to another embodiment, the generating of a series of robot instruction information or a series of trainee instruction information may further include: generating movement instruction to change a location of the robot; and generating a gesture instruction to change a motion of the robot.

According to another embodiment, the generating of a series of robot instruction information or a series of trainee instruction information may further include: determining whether the trainee is already registered by using the collected trainee color information; and registering the trainee information.

According to another embodiment, the generating of trainee behavior information based on the sensor information collected by the sensor unit may further include: dividing an available place of the trainee into a plurality of spaces; and generating the trainee behavior information by matching three-dimensional location information of the trainee with the plurality of spaces.

According to another embodiment, the generating of trainee behavior information based on the sensor information collected by the sensor unit may further include calculating a bending angle of a physical joint of the trainee based on the three-dimensional location information of the trainee.

According to another embodiment, there is also provided a system for training recognition ability, which includes: an apparatus for training recognition ability including an instruction generating unit for generating a series of robot instruction information to instruct a behavior of a robot or a series of trainee instruction information to instruct a behavior of a trainee, a sensor unit for collecting three-dimensional location information of the trainee who imitates the behavior of the robot according to the robot instruction information or acts according to the trainee instruction information, and sensor information including color information or mobile device usage information of the trainee, a trainee behavior information generating unit for generating trainee behavior information based on the sensor information collected by the sensor unit, and a recognition ability determining unit for calculating recognition ability of the trainee based on the trainee behavior information along with the robot instruction information or the trainee instruction information; a robot communicating with the apparatus for training recognition ability to operate according to the series of robot instruction information; and a mobile device used by the trainee and to which information is input according to the trainee instruction information.

### [Advantageous Effects]

According to an aspect of the present disclosure, recognition ability of a patent may be treated in a three-dimensional real space by delivering a behavior instruction to the patient by means of a robot.

According to another aspect of the present disclosure, recognition ability may be improved more in comparison to a conventional recognition treatment since the whole body of a patient may be utilized.

According to another aspect of the present disclosure, space perception ability, short term memory ability, eyesight or the like of a patent may be developed.

### [Description of Drawings]

Fig. 1 is a diagram showing a recognition ability training apparatus 100, robot 200 and trainee 300 and a mobile device 400 used by a trainee according to an embodiment of the present disclosure.
Fig. 2 is a diagram showing a configuration of the recognition ability training apparatus 100 according to an embodiment of the present disclosure.
Fig. 3 is a diagram showing a configuration of an instruction generating unit 110 according to an embodiment of the present disclosure.
Fig. 4 is a diagram showing a configuration of a sensor unit 120 according to an embodiment of the present disclosure.
Fig. 5 is a diagram showing a configuration of a trainee information managing unit 130 according to an embodiment of the present disclosure.
Fig. 6 is a diagram showing a configuration of a trainee behavior information generating unit 140 according to an embodiment of the present disclosure.
Fig. 7 is a diagram showing an activity place of a robot and a trainee moving according to a movement instruction according to an embodiment of the present disclosure.
Fig. 8 is a diagram showing a configuration of a trainee gesture information processing unit 142 according to an embodiment of the present disclosure.
Fig. 9 is a diagram for expressing a body of the trainee with joints according to an embodiment of the present disclosure.
Fig. 10 is a diagram showing a configuration of a robot 200 according to an embodiment of the present disclosure.
Fig. 11 is a flowchart for illustrating a method for evaluating recognition ability according to an embodiment of the present disclosure.

### [Mode for Invention]

The present disclosure will be described in detail based on specific embodiments for implementing the present disclosure with reference to the accompanying drawings. There embodiments will be fully described so that a person having ordinary skill in the art can easily implement the present disclosure. Various embodiments of the present disclosure should be understood not to exclude each other even though they are different from each other. For example, specific shapes, structures and features of an embodiment described herein may be implemented in other embodiments without departing from the spirit or scope of the present disclosure. In addition, it should be understood that locations and arrangements of individual components of an embodiment disclosed herein can be modified without departing from the spirit or scope of the present disclosure. Therefore, the following detailed description is not to limit the present disclosure, and if appropriately explained, the scope of the present disclosure will be defined only by the appended claims along with equivalents thereof. In the drawings, like reference numerals denote like elements in various aspects.

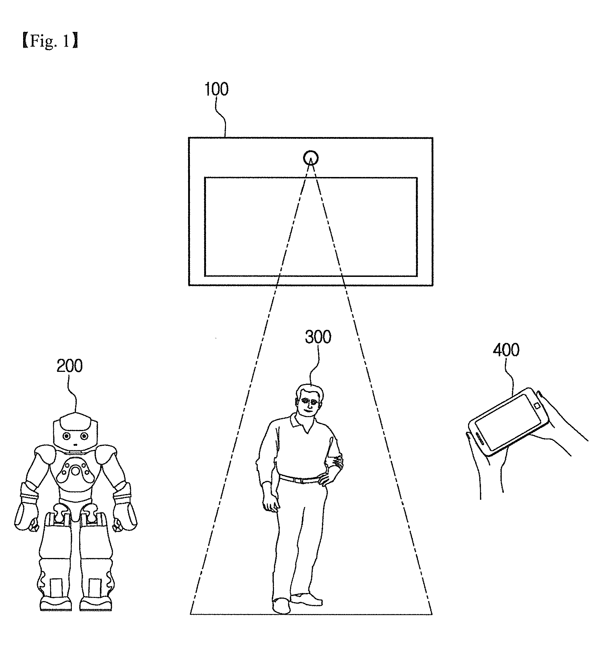

Fig. 1 is a diagram showing a recognition ability training apparatus 100, a robot 200, a trainee 300 and a mobile device 400 used by the trainee. The recognition ability training apparatus 100 plays a role of generating robot instruction information to control a behavior of the robot 200 or a series of trainee instruction information to control a behavior of the trainee, collecting information of the trainee 300 who acts according to the information, comparing the robot instruction information or the trainee instruction information with trainee information, and training recognition ability of the trainee according to the comparison result. The recognition ability training apparatus 100 of Fig. 1 checks through a sensor a location of the trainee 300 and whether various gestures and mobile devices are used. The recognition ability training apparatus 100 will be described in detail later.

The robot 200 receives the robot instruction information of the recognition ability training apparatus 100 and performs various behaviors. In an embodiment, the robot 200 may have an appearance and form similar to a human and may also have two arms to manipulate something by hands. In other words, the robot 200 may be a humanoid robot and may also have a plurality of joints. The humanoid robot may change a posture of arms or legs by adjusting each joint. In addition, the humanoid robot may walk, throw an article or kick a ball. In another embodiment, the robot 200 may have a humanoid configuration only at a part of the robot components. For example, an upper body of the robot may have a human appearance, but wheels may be mounted to a lower body thereof for easier movement.

The trainee 300 watches robot instruction information expressed by the robot 200 and imitates the expression. The trainee may be a person having a disorder in recognition ability or a person who desires the improvement of recognition ability. The trainee is not particularly limited. In addition, the trainee 300 performs a behavior according to the trainee instruction information. The trainee may imitate a motion or gesture transferred through a display unit or a voice unit, and may also manipulate the robot or give an answer to a quiz.

The mobile device 400 is used for expressing the trainee instruction transmitted from the recognition ability training apparatus 100. The mobile device 400 is used by the trainee 300. In an embodiment, the trainee instruction may be an instruction for making a motion, imitating a gesture or moving the robot 300, and may also be an action for manipulating the robot or giving an answer to a quiz. These behaviors may be expressed through various kinds of inputs such as touch input or tilting input of the mobile device 400. The mobile device 400 may be implemented in various ways and may include various features. For example, the mobile device 100 may be processing devices such as a cellular device, a personal digital assistant (PDA), a digital camera, a digital camera-enabled mobile phone, a portable computer or the like. In particular, the mobile device 400 may be a smart phone or a small smart pad including a display, a touch sensor, a motion sensor, an oscillator, a speaker, a communication module or the like. In addition, the mobile device 400 may include a processing system for allowing communication between at least one software application and an operating system by being equipped with a processor, an operating system and an application program interface (API). Further, the processing system of the mobile device 400 may be configured to execute various software applications. The mobile device 400 may communicate with the recognition ability training apparatus 100 or another external device, and any hardware or software for communication may be loaded therein. All kinds of transmittable information such as various sensor information in the mobile device 400 may be transmitted to or received from the recognition ability training apparatus 400 or other external device (not shown) through the mobile device 400. The communication method may be WiFi or BlueTooth, without being limited thereto. The sensor unit of the mobile device 400 senses information about various external inputs and may include a touch sensor, an acceleration sensor, a magnetic sensor and a rotation sensor. The touch sensor plays a role of sensing a touch input of a user to the mobile device 400. The touch sensor may sense not only a single touch but also a multi touch. The pattern of the touch input may be a location of a touched point, or a state of a point such as a new point, a moved point or a released point, or several touching gestures such as tab, double tab, panning, flicking, drag-and-drop, pinching and stretching. The acceleration sensor may measure an acceleration applied according to the movement of the mobile device 400 or the gravity. The magnetic sensor may measure an intensity of a magnetic field around the mobile device 400. The rotation sensor is a sensor for recognizing three-dimensional movement of the mobile device 400 and is also called a gyro sensor. The rotation sensor may have only two axes or three axes. In an embodiment, each sensor may be a three-axis sensor, but this may also be degenerated into a one-axis sensor or a two-axis sensor. In addition, each sensor may be separated for each axis.

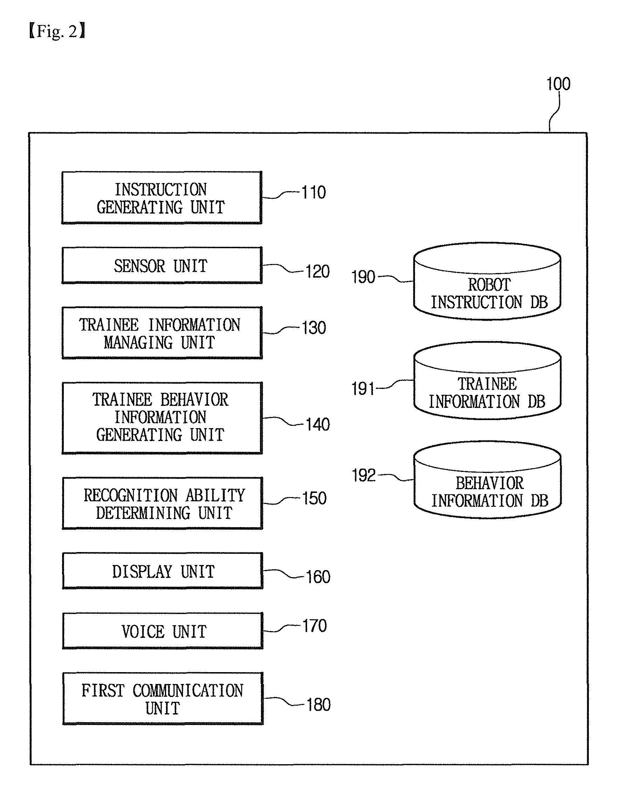

Fig. 2 shows a configuration of the recognition ability training apparatus 100. The recognition ability training apparatus 100 may include an instruction generating unit 110, a sensor unit 120, a trainee information managing unit 130, a trainee behavior information generating unit 140, a recognition ability determining unit 150, a display unit 160, a voice unit 170, a first communication unit 180, a robot instruction DB 190, a trainee information DB 191, and a behavior information DB 192.

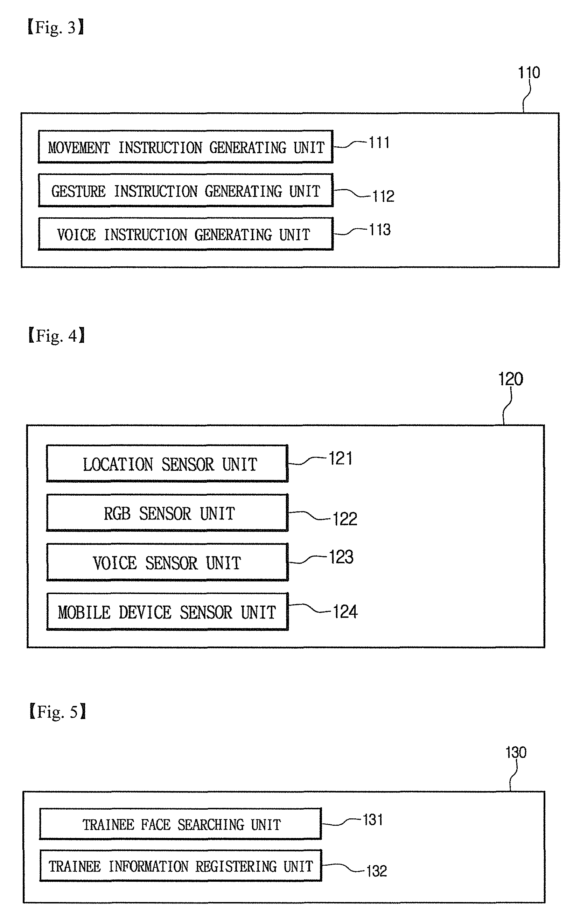

The instruction generating unit 110 plays a role of generating a series of robot instruction information to control a behavior of the robot or a series of trainee instruction information to instruct a behavior of the trainee. The robot instruction information is a command or instruction for controlling a behavior of the robot 200 and may represent all behaviors which may be performed by the robot. The trainee instruction information represents all behaviors which may be performed by the trainee, and for example, the trainee may give an answer to a quiz expressed by the display unit or the voice unit of the recognition ability training apparatus 100, imitate a behavior expressed thereby or manipulate the robot. Without being limited thereto, numerous behaviors which may be performed by the trainee may be expressed. In an embodiment, the level of difficulty of the robot instruction information may be adjusted by changing the configuration of the series of robot instruction information or trainee instruction information. The number of successive instruction information may be increased, various kinds of instruction information may be mixed, and the time limit during which the instruction information should be imitated may be adjusted. The level of difficulty may be adjusted in various ways without being limited to the above. In another embodiment, the instruction generating unit 110 generates a series of robot instruction information or trainee instruction information based on the trainee information, and the trainee information may include face color information, gender information and age information of the trainee and usage information of the recognition ability training apparatus. In other words, by generating easy or difficulty instruction information according to the level of the trainee or generating instruction information other than the instruction information which has been already expressed to the trainee, instruction information may be generated to improve the recognition ability more efficiently. For example, if the trainee is 70 or above years old, the level of generated instruction may be lowered, and if the trainee has scored a point over a certain level for any instruction information, more difficult instruction information may be generated. The instruction generating unit may include, as shown in Fig. 3, a movement instruction generating unit 111 for generating movement instruction information to change a location of the robot 200, a gesture instruction generating unit 112 for generating gesture instruction information to change a behavior of the robot 200, and a voice instruction generating unit 113 for transmitting an instruction in voice. Although not shown in the figures, an instruction generating unit for directly requesting various behaviors to the trainee through the voice unit or the display unit may also be provided.

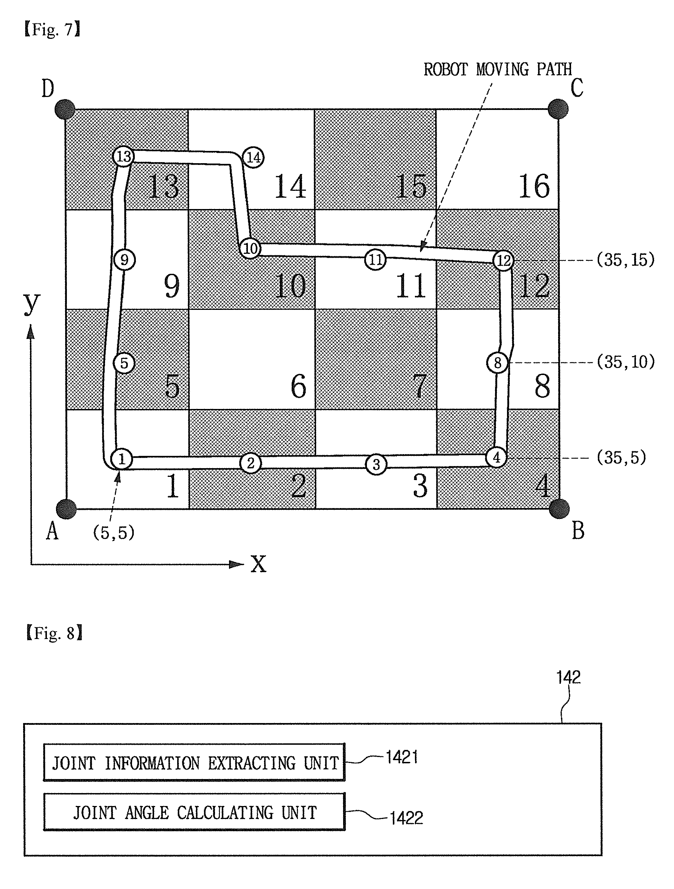

The movement instruction generating unit 111 may generate a series of movement instruction information for subsequent movement in the order of 4→8→12→11→10→14→13→9→5→1→2→3 in a rectangular space as shown in Fig. 7. In another embodiment, the movement instruction generating unit 111 may generate movement instruction information for movement to the left, movement to the right, movement to the front, and movement to the rear. The movement instruction generating unit 111 may store relative coordinates from the recognition ability training apparatus 100, absolute coordinates of the robot in a space where the robot is moving, or space information where the robot is moving (for example, the space of 4→8→12→11→10→14→13→9→5→1→2→3) in the robot instruction DB 190 as robot instruction information.

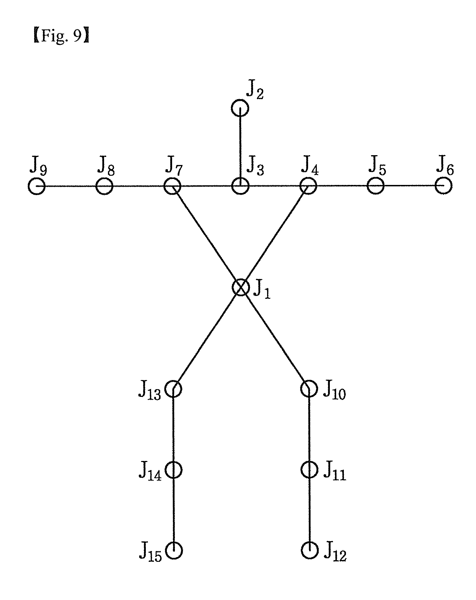

The gesture instruction generating unit 112 may generate gesture instruction information for the robot 200 to stretch out the right and left arms to the front, to the rear, to the side, to the above, to the below or the like. In addition, the gesture instruction generating unit 112 may also generate gesture instruction information for sitting down and then standing up, jumping, turning, clapping or the like. In addition, the gesture instruction generating unit 112 may also generate gesture instruction information using the head in relation to a so-called head gesture for shaking the head laterally, shaking the head vertically, tilting the head or the like. The gesture instruction generating unit 112 may also generate gesture instruction information using the face in relation to a so-called look gesture for closing the eyes, smiling, frowning, being startled or the like. The gesture instruction generating unit 112 may store angles of all or some joints of the robot 200 in the robot instruction DB 190 as robot instruction information. The location of each joint of the robot 200 and its name may be expressed as in Table 1 below with reference to Fig. 9.

**[Table 1]**

| Joint location | Joint name |
|---|---|
| J1 | torso (TOR) |
| J2 | head (HEAD) |
| J3 | neck (NECK) |
| J4 | Left shoulder (LS) |
| J5 | Left elbow (LE) |
| J6 | Left hand (LW) |
| J7 | Right shoulder (RS) |
| J8 | Right elbow (RE) |
| J9 | Right hand (RW) |
| J10 | Left hip (LH) |
| J11 | Left knee (LK) |
| J12 | Left foot (LF) |
| J13 | Right hip (RH) |
| J14 | Right knee (RK) |
| J15 | Right foot (RF) |

Table 2 shows a method for storing angles of all or some joints of the robot 200 in the robot instruction DB 190. For example, J_{7_8} represents a straight line between J₇ and J₈, and J_{7_8}-J_{7_3} represents an angle between both lines. Joint angles of all joints or related to a specific gesture may be stored as robot instruction information in the robot instruction DB 190.

**[Table 2]**

| gesture instruction information | robot joint angle 1 | robot joint angle 2 |
|---|---|---|
| Right hand to the front | J_{7_8}-J_{7_3}=90° | J_{4_3}-J_{4_5}=90° |
| | J_{7_8}-J_{7_1}=90° | J_{4_5}-J_{4_1}=135° |
| Left hand to the above | | |
| | ··· | ··· |
| Right hand to the front | J_{7_8}-J_{7_3}=90° | J_{4_3}-J_{4_5}=90° |
| | J_{7_8}-J_{7_1}=90° | J_{4_5}-J_{4_1}=90° |
| Left hand to the front | | |
| | ··· | ··· |
| Bending the knee | J_{14_13}-J_{14_15}<90° | J_{11_10}-J_{11_12}<90° |
| | ··· | ··· |

The voice instruction generating unit 113 generates voice instruction information for the robot 200 to express a gesture such as push-up, knee turning, kick by a right leg, kick by a left leg or the like. In an embodiment, an item which may not be easily expressed by the movement or gesture instruction information of the robot may be expressed using the voice instruction information, but the movement or gesture instruction information of the robot may also be generated using the voice instruction information. The voice instruction may also be stored as robot instruction information in the robot instruction DB 190.

The instruction generating unit 110 may transmit the robot instruction information by means of at least one communication method selected from the group consisting of LAN (Local Area Network), MAN (Metropolitan Area Network), GSM (Global System for Mobile Network), EDGE (Enhanced Data GSM Environment), HSDPA (High Speed Downlink Packet Access), W-CDMA (Wideband Code Division Multiple Access), CDMA (Code Division Multiple Access), TDMA (Time Division Multiple Access), Bluetooth, Zigbee, Wi-Fi, VoIP (Voice over Internet Protocol), Wi-MAX (World Interoperability for Microwave Access) and ultrasonic communication. However, in some embodiments, the communication may be separately performed by the first communication unit 180.

The sensor unit 120 plays a role of collecting three-dimensional location information of the trainee who imitates a behavior of the robot according to the robot instruction information or acts according to the trainee instruction information and sensor information including color information or mobile device usage information. In the recognition ability training apparatus 100 of Fig. 1, a portion expressed by a circle represents a part of the sensor unit 120, which plays a role of collecting information of the trainee 300. As shown in Fig. 4, the sensor unit 120 may include a location sensor unit 121 for collecting three-dimensional location information of the trainee 300, a RGB sensor unit 122 for collecting color information of the trainee, a voice sensor unit 123 for collecting sounds made by the trainee, and a mobile device sensor unit 124 for collecting mobile device usage information of the trainee.

In an embodiment, the location sensor unit 121 may be a depth sensor, which may be configured with an infrared laser projector coupled to a CMOS and sense three-dimensional images in any brightness condition by using infrared rays emitted by a single camera and reflected by an object. The depth sensor may sense not only horizontal and vertical directions but also a proximal or distal distance to check a location and behavior of the whole body. In order to obtain a depth image by an infrared camera, the time-of-flight method is generally used, but a depth may also be calculated by means of stereo matching after a pattern (a structured light) is projected to a target. In another embodiment, the trainee may be detected by checking a moving article having a stepped distance by means of blob labeling in a stream sequence to chase a location in a space, and an ID may be endowed to each moving article.

In an embodiment, the RGB sensor unit 122 collects the color information of a subject, the trainee, by using three colors of red, green and blue. In an embodiment, when color information of the face is collected, this may be used to determine whether the trainee is already registered or not.

In an embodiment, the mobile device sensor unit 124 plays a role of collecting information from a touch sensor, an acceleration sensor, a magnetic sensor and a rotation sensor in the mobile device 400.

The trainee information managing unit 130 determines whether the trainee is already registered by using the color information of the trainee, and registers the trainee information. As shown in Fig. 5, the trainee information managing unit 130 may include a trainee face searching unit 131 for searching trainee information of a registered trainee which is identical to the face color information among the color information of the trainee, and a trainee information registering unit 132 for registering trainee information including the face color information among the collected color information of the trainee if a face identical to the face color information is not searched by the trainee face searching unit 131. In an embodiment, based on the face color information of the trainee, the face of a trainee may be searched or compared with reference to human face feature information. Here, the human face feature information is reference data which generally expresses shapes of eyebrows, eyes, a nose, eyes or the like into a specific pattern so that a face may be easily recognized from color information extracted therefrom. In an embodiment, the trainee face searching unit 131 may search the trainee information from the trainee information DB 191. In another embodiment, a face may also be searched by using a local binary pattern and an Adoboost technique. In another embodiment, when the trainee information is registered, gender and age of the trainee may be estimated from the face information and stored as the trainee information, and information about gender or age may be directly input by the trainee 300 through the display unit 160 or the voice unit 170. This may also be utilized as data which is referred to when the instruction generating unit 110 generates robot instruction information.

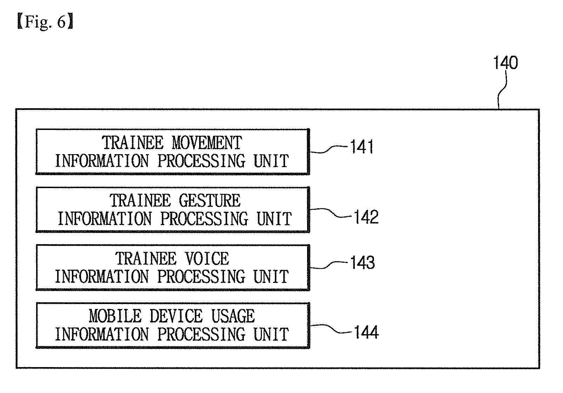

The trainee behavior information generating unit 140 plays a role of generating trainee behavior information based on the collected sensor information. As shown in Fig. 6, the trainee behavior information generating unit 140 may include a trainee movement information processing unit 141 for processing the trainee movement information collected by the sensor, a trainee gesture information processing unit 142 for processing gesture information, a trainee voice information processing unit 143 for processing voice information, and a mobile device usage information processing unit 144 for processing mobile device usage information.

In an embodiment, the trainee movement information processing unit 141 generates trainee behavior information by processing the trainee movement information based on the location information collected by the sensor unit 120. First, as shown in Fig. 7, a space where the trainee or the robot is moving is divided into a plurality regions. In Fig. 7, the space is divided into 16 regions, four in a horizontal direction and four in a vertical direction, and while setting the horizontal direction as an X axis and the vertical direction as a Y axis, each region is allocated with a size of 10x10. Even though the space is divided into square regions for easy explanation, the size of each divided region may be adjusted as desired. The trainee movement information processing unit 141 checks a XY coordinate where a center of gravity of the trainee is located, namely a region corresponding to the location information among 16 divided regions in real time and allocates a region to the coordinate. In this case, the trainee behavior information is space information allocated to the movement of the center of gravity. The center of gravity may be calculated by means of a K-means algorithm, which is a clustering technique based on a distance by which an aggregation of location information for the whole body of the trainee is classified into the K number of groups. When a series of movement instruction information such as a movement in the order of 4→8→12→11→10→14→13→9→5→1→2→3 in the space of Fig. 7 is expressed by the robot 200, the trainee may watch and memorize the movement and check a moving path through the regions allocated according to the movement of the center of gravity.

In an embodiment, the trainee gesture information processing unit 142 generates trainee behavior information by processing trainee gesture information based on the location information collected by the sensor unit 120. Joint information may be extracted from the location information of the trainee, and a gesture of the trainee may be checked by calculating a bending angle of each joint. In this case, the trainee behavior information may be a bending angle of each joint.

In an embodiment, the mobile device usage information processing unit 144 generates trainee behavior information by utilizing the mobile device usage information collected through the sensor unit 120. A behavior of the trainee may be checked through a mobile device by checking the change of touch, acceleration, magnetic field and rotation sensor values of the mobile device 400. In this case, the trainee behavior information may be a behavior of the trainee checked through the mobile device.

The recognition ability determining unit 150 plays a role of calculating recognition ability of the trainee based on the robot instruction information or the trainee instruction information and the trainee behavior information. In other words, the recognition ability of the trainee is measured by checking whether the trainee accurately memorizes the robot instruction information or the trainee instruction information in regular order through the comparison with the trainee behavior.

In an embodiment, if the robot instruction information is movement instruction information for changing a location of the robot, the robot instruction information including a relative coordinate from the recognition ability training apparatus 100 to the robot, an absolute coordinate of the space where the robot is moving and space information where the robot is moving is compared with the trainee behavior information including a relative coordinate from the recognition ability training apparatus 100 to the center of gravity of the trainee, an absolute of the center of gravity of the trainee or space information allocated to the center of gravity of the trainee. When the identity is checked by means of absolute coordinates or relative coordinates, it is assumed that they are in the same space if each coordinate is a coordinate in a divided space region. In case of a series of successive robot instruction information, a ratio of the number of all robot instruction information to the number of identical robot instruction information may be set as a recognition ability point. For example, in case of the robot instruction information which moves through 12 space regions of 4→8→12→11→10→14→13→9→5→1→2→3 as shown in Fig. 7, if the trainee moves through the space regions of 4→8→12→11→15→14→13→9→5→7→2→3, ten space regions are identical, which corresponds to a ratio of 10/12 and may be calculated as 83 recognition ability points in percentage. The calculated recognition ability point may be expressed through the display unit 160. In addition, the point may be notified to the trainee through the voice unit 170. In a specific embodiment, when any movement instruction information is identical, the voice unit 170 may instantly notify this to the trainee in voice.

In another embodiment, if the robot instruction information is gesture instruction information for changing a behavior of the robot, the robot instruction information storing all joint angles of the robot or a joint angle in relation to a specific gesture is compared with the trainee behavior information storing a bending angle of each joint extracted from the location information of the trainee. If the difference of joint angles between the robot instruction information and the trainee behavior information is within a predetermined range (for example, 10 degrees), it is determined that the same gesture is performed and then the recognition ability point may be calculated. As in the embodiment of the movement instruction information, the identity may be measured for each of a series of gesture instruction information to calculate the recognition ability point. In addition, the calculated recognition ability point may be displayed through the display unit 160. Moreover, the point may be notified to the trainee through the voice unit 170. In a specific embodiment, if the movement instruction information is identical, the voice unit 170 may instantly notify the same to the trainee in voice.

In another embodiment, if the robot instruction information or the trainee instruction information and the trainee behavior information are determined as being identical within a predetermined range, the instruction generating unit generates robot instruction information for controlling the behavior of the robot again so that the robot performs a feedback behavior, or provide the feedback information to the trainee through the display unit or the voice unit. In other words, a configuration for directly delivering the information notifying an imitating behavior of the trainee is identical to the robot instruction information to the trainee in real time is implemented through the robot. The robot instruction information generated again may include all of general robot instruction information, and in an embodiment, this may be voice instruction information for delivering voice information such as "clap, clap, clap", "you got it", "OK" or the like through the robot. The robot instruction information may also be gesture information for allowing the robot to actually clap or for allowing the robot to make a smiling or grimacing look. By doing so, the trainee may check whether his behavior is identical to the robot instruction information, and the recognition ability may be evaluated more accurately by reinforcing the interaction with the robot. In addition, the information notifying that the behavior of the trainee is identical to the robot instruction information or the trainee instruction information may be directly transferred to the trainee through the display unit or the voice unit of the recognition ability training apparatus 100.

The display unit 160 plays a role of displaying various kinds of information provided from the recognition ability training apparatus 100 to the trainee. The trainee information and the recognition ability point may be displayed through the display unit 160, and the trainee information may be input therethrough. The display unit 160 may be a LCD (Liquid Crystal Display), a PDP (Plasma Display Panel), or a projector display, and this may also be a three-dimensional display using autostereography or hologram in a shutter glass manner, a lenticular manner, a parallax barrier manner or the like or a touch screen display capable of recognizing a touch input.

The voice unit 170 plays a role of delivering various kinds of information provided from the recognition ability training apparatus 100 to the trainee in voice and includes various sound modules capable of generating voices. In an embodiment, the voice unit 170 may guide an imitating time limit of the robot instruction information to the trainee and adjust an interval of the imitating time limit. For example, when a series of moving gesture information for moving to the space regions of 4→8→12→11→10→14→13→9→5→1→2→3 of Fig. 7 is generated, a time for the trainee to perform a behavior is guided. An interval for moving to each space region may be set to be three seconds, and if the trainee does not move to a next space region within three seconds, the trainee behavior information generating unit may determine that the trainee behavior information is not identical to the robot instruction information even when the trainee moved to the corresponding space region after three seconds. In addition, the level of difficulty of the series of robot instruction information may be adjusted by changing the interval of the imitating time limit. If the interval is set to be 1.5 seconds, the trainee should perform a behavior within a shorter time, in comparison to the case where the interval is set to be 3 seconds, and thus the recognition ability may be evaluated in a more difficult level even though the same robot instruction information is used.

The first communication unit 180 may perform communication by means of at least one communication method selected from the group consisting of wireless LAN (Local Area Network), MAN (Metropolitan Area Network), GSM (Global System for Mobile Network), EDGE (Enhanced Data GSM Environment), HSDPA (High Speed Downlink Packet Access), W-CDMA (Wideband Code Division Multiple Access), CDMA (Code Division Multiple Access), TDMA (Time Division Multiple Access), Bluetooth, Zigbee, Wi-Fi, VoIP (Voice over Internet Protocol), Wi-MAX (World Interoperability for Microwave Access) and ultrasonic communication. However, in some embodiments, the first communication unit 180 may not be separately provided but be included as a single function of another component of the recognition ability training apparatus 100. For example, the robot instruction information of the instruction generating unit 110 may be directly transmitted to the robot 200 at the outside.

The robot instruction DB 190 plays a role of storing the robot instruction information generated by the instruction generating unit 110. In an embodiment, a relative coordinate from the recognition ability training apparatus 100, an absolute coordinate of the space where the robot is moving and angles of all or some joints of the robot 200 may be stored in the robot instruction DB 190. In another embodiment, angles of all or some joints of the robot 200 may be stored in the robot instruction DB 190.

The trainee information DB 191 stores information in relation to the trainee using the recognition ability training apparatus 100, namely the trainee information. The trainee information may include face color information, gender information, age information, usage information of the recognition ability training apparatus, and in case of a new trainee, the trainee information is stored by the trainee information managing unit 140.

The behavior information DB 192 stores behavior information of the trainee. The behavior information of the trainee is generated based on the collected sensor information and may be coordinate information, space information, joint angle information or the like as described above.

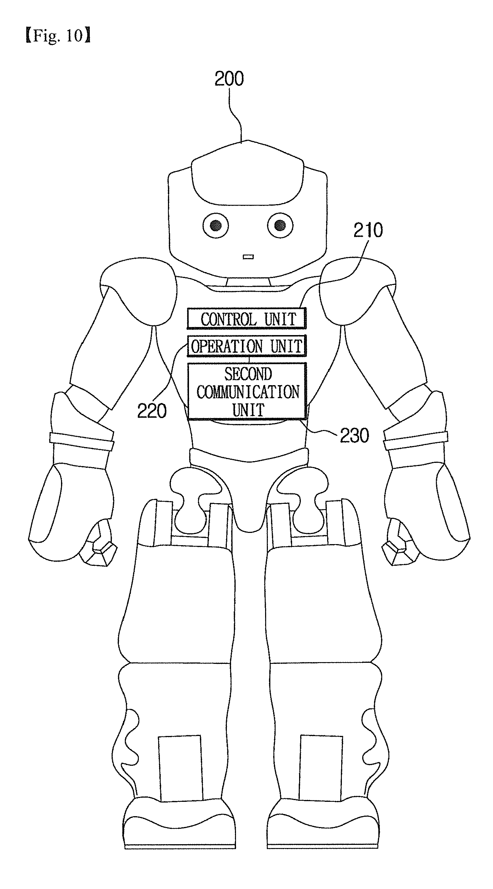

Fig. 10 shows a configuration of the robot 200. In an embodiment, the robot 200 may be a humanoid as described above, or a part of the robot 200 may be configured as a humanoid. The robot 200 may include a control unit 210, an operation unit 220, and a second communication unit 230. The robot 200 receives the robot instruction information from the recognition ability training apparatus 100 through the second communication unit 230, controls the operation unit 220 by means of the control unit 210, and changes a location or behavior of the robot 200 according to the robot instruction information. In addition, the robot 200 may deliver the voice information to the trainee through a voice unit (not shown). The second communication unit 230 may perform communication in the same way as the recognition ability training apparatus 100.

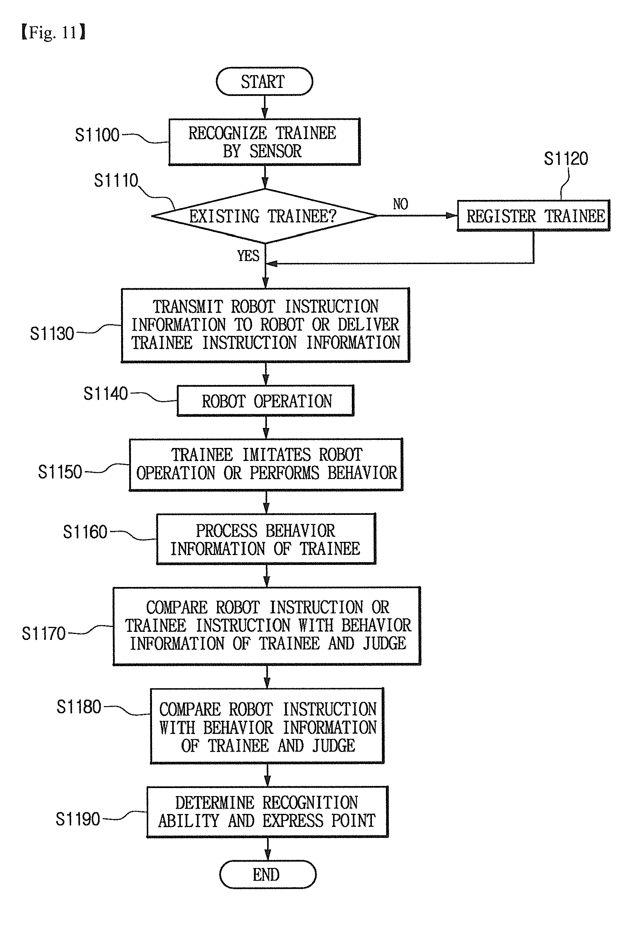

Fig. 11 is a flowchart for illustrating a method for evaluating recognition ability. First, the recognition ability training apparatus 100 recognizes a trainee by using a sensor function (S1100). In an embodiment, the trainee is recognized by using color information, particularly face color information, obtained by a RGB camera. In an embodiment, face feature information about eyes, a nose, a mouth and eyebrows may be referred to. If the recognized trainee is not an existing trainee (S1110), for example, if the recognized face information does not fall within face information expression values of an existing trainee, the trainee is newly registered (S1120). In this case, additional information may be input from the trainee, and additional information such as gender and age may be estimated from the recognized face color information of the trainee. If the trainee is registered or recognized as an existing trainee, the recognition ability training apparatus 100 transmits the robot instruction information to the robot 200 or delivers the trainee instruction information to the trainer. As described above, the robot instruction information may include movement instruction information, gesture instruction information, and voice instruction information, without being limited thereto. The trainee instruction information includes an instruction for manipulating the robot, without being limited thereto. The robot operates according to the given robot instruction information. In case of the trainee instruction, the process for operating the robot is excluded. However, in case of the instruction for manipulating the robot, the robot may operate by the robot instruction generated or transmitted by the recognition ability training apparatus according to an input of the trainee through the mobile device (S1130). The trainee watches and imitates various kinds of robot instruction information of the robot or acts according to the trainee instruction (S1140). By memorizing and imitating a series of robot instruction information or trainee instruction information, the trainee may improve the recognition ability, particularly the power of memory. While the trainee performs a behavior, the recognition ability training apparatus 100 recognizes the behavior information of the whole body of the trainee by means of sensors or the usage of the mobile device (S1160). By means of the location information recognized by the sensor and the sensor information including the color information, the behavior information is processed to check a behavior of the trainee (S1170). After that, the robot instruction information or the trainee instruction information is compared with the behavior information of the trainee to check identity between them (S1180), and based on this, the recognition ability is judged and its point is expressed (S1 190). In this case, this result may be fed back in real time as voice or visual information through the robot 200 or the recognition ability training apparatus 100.

While the exemplary embodiments have been shown and described, it will be understood by those skilled in the art that various changes in form and details may be made thereto without departing from the spirit and scope of the present disclosure as defined by the appended claims. In addition, many modifications can be made to adapt a particular situation or material to the teachings of the present disclosure without departing from the essential scope thereof.

Therefore, it is intended that the present disclosure not be limited to the particular exemplary embodiments disclosed as the best mode contemplated for carrying out the present disclosure, but that the present disclosure will include all embodiments falling within the scope of the appended claims.

### [Industrial Applicability]

According to an aspect of the present disclosure, recognition ability of a patent may be treated in a three-dimensional real space by delivering a behavior instruction to the patient by means of a robot.

According to another aspect of the present disclosure, recognition ability may be improved more in comparison to a general recognition treatment since the whole body of a patient may be utilized.

According to another aspect of the present disclosure, space perception ability, short term memory ability, eyesight or the like of a patent may be developed.

## Claims

1. An apparatus for training recognition ability, comprising:
an instruction generating unit for generating a series of robot instruction information to instruct a behavior of a robot or a series of trainee instruction information to instruct a behavior of a trainee;
a sensor unit for collecting three-dimensional location information of the trainee who imitates the behavior of the robot according to the robot instruction information or acts according to the trainee instruction information, and sensor information comprising color information or mobile device usage information of the trainee;
a trainee behavior information generating unit for generating trainee behavior information based on the sensor information collected by the sensor unit; and
a recognition ability determining unit for calculating recognition ability of the trainee based on the trainee behavior information along with the robot instruction information or the trainee instruction information.

2. The apparatus for training recognition ability according to claim 1, further comprising:
a first communication unit capable of communicating with an arbitrary object external to the apparatus for training recognition ability;
a display unit for providing visual information to the trainee; and
a voice unit for providing voice information to the trainee,
wherein the series of robot instruction information is transmitted to the robot through the first communication unit, and the trainee instruction information is delivered to the trainee through the display unit or the voice unit.

3. The apparatus for training recognition ability according to claim 1,
wherein the mobile device usage information is generated from at least one of a touch sensor, an acceleration sensor, a magnetic sensor and a rotation sensor in the mobile device, and
wherein when the trainee instruction information is an instruction for operating the robot by using the mobile device, the instruction generating unit generates a series of robot instruction information again.

4. The apparatus for training recognition ability according to claim 1,
wherein when it is determined that the robot instruction information or the trainee instruction information is identical to the trainee behavior information within a predetermined range, the instruction generating unit generates robot instruction information for controlling the behavior of the robot again so that the robot performs a feedback behavior or feedback information is provided to the trainee through the display unit or the voice unit.

5. The apparatus for training recognition ability according to claim 2,
wherein the voice information guides a time limit for the trainee to imitate the robot instruction information or act according to the trainee instruction information, the time limit being adjustable.

6. The apparatus for training recognition ability according to claim 1,
wherein the instruction generating unit generates the series of robot instruction information or trainee instruction information based on trainee information, and
wherein the trainee information comprises face color information, gender information and age information of the trainee, and usage information of the apparatus for training recognition ability.

7. The apparatus for training recognition ability according to claim 1, wherein the instruction generating unit comprises:
a movement instruction generating unit for generating movement instruction to change a location of the robot; and
a gesture instruction generating unit for generating a gesture instruction to change a motion of the robot.

8. The apparatus for training recognition ability according to claim 1, wherein the sensor unit comprises:
a location sensor unit for collecting three-dimensional location information of the trainee;
a red-green-blue (RGB) sensor unit for collecting color information of the trainee; and
a mobile device sensor unit for collecting mobile device usage information of the trainee.

9. The apparatus for training recognition ability according to claim 6, further comprising a trainee information managing unit for determining whether the trainee is already registered by using the collected color information of the trainee and registering the trainee information.

10. The apparatus for training recognition ability according to claim 8,
wherein the trainee information managing unit comprises:
a trainee face searching unit for searching corresponding trainee information from pre-stored trainee information based on face color information among the collected color information of the trainee; and
a trainee information registering unit for registering trainee information comprising the face color information among the collected color information of the trainee, if a corresponding face is not searched by the trainee face searching unit.

11. The apparatus for training recognition ability according to claim 6,
wherein the trainee behavior information generating unit divides an available place of the trainee into a plurality of spaces, and generates the trainee behavior information by matching three-dimensional location information of the trainee with the plurality of spaces.

12. The apparatus for training recognition ability according to claim 6,
wherein the trainee behavior information generating unit generates the trainee behavior information by calculating a bending angle of a physical joint of the trainee based on the three-dimensional location information of the trainee.

13. A method for training recognition ability, comprising:
generating a series of robot instruction information to instruct a behavior of a robot or a series of trainee instruction information to instruct a behavior of a trainee;
collecting three-dimensional location information of the trainee who imitates the behavior of the robot according to the robot instruction information or acts according to the trainee instruction information, and sensor information comprising color information or mobile device usage information of the trainee;
generating trainee behavior information based on the sensor information collected by the sensor unit; and
calculating recognition ability of the trainee based on the trainee behavior information along with the robot instruction information or the trainee instruction information.

14. The method for training recognition ability according to claim 13, wherein said generating a series of robot instruction information or a series of trainee instruction information further comprises:
transmitting the series of robot instruction information to the robot; and
delivering the trainee instruction information to the trainee as visual information through a display unit or voice information through a voice unit.

15. The method for training recognition ability according to claim 13, wherein said generating a series of robot instruction information or a series of trainee instruction information further comprises:
generating a series of robot instruction information again when the trainee instruction information is an instruction for operating the robot by using the mobile device.

16. The method for training recognition ability according to claim 13, wherein, in said calculating of recognition ability of the trainee based on the trainee behavior information along with the robot instruction information or the trainee instruction information, when it is determined that the robot instruction information or the trainee instruction information is identical to the trainee behavior information within a predetermined range, robot instruction information for controlling the behavior of the robot is generated again so that the robot performs a feedback behavior or feedback information is provided to the trainee through the display unit or the voice unit.

17. The method for training recognition ability according to claim 14,
wherein the voice information guides a time limit for the trainee to imitate the robot instruction information or act according to the trainee instruction information, the time limit being adjustable.

18. The method for training recognition ability according to claim 13,
wherein said generating of a series of robot instruction information or a series of trainee instruction information further comprises generating the series of robot instruction information or trainee instruction information based on trainee information, and
wherein the trainee information comprises face color information, gender information and age information of the trainee, and usage information of the apparatus for training recognition ability.

19. The method for training recognition ability according to claim 13, wherein said generating of a series of robot instruction information or a series of trainee instruction information further comprises:
generating movement instruction to change a location of the robot; and
generating a gesture instruction to change a motion of the robot.

20. The method for training recognition ability according to claim 18, wherein said generating of a series of robot instruction information or a series of trainee instruction information further comprises:
determining whether the trainee is already registered by using the collected color information of the trainee; and
registering the trainee information.

21. The method for training recognition ability according to claim 13, wherein said generating of trainee behavior information based on the sensor information collected by the sensor unit further comprises:
dividing an available place of the trainee into a plurality of spaces; and
generating the trainee behavior information by matching three-dimensional location information of the trainee with the plurality of spaces.

22. The method for training recognition ability according to claim 13, wherein said generating of trainee behavior information based on the sensor information collected by the sensor unit further comprises:
calculating a bending angle of a physical joint of the trainee based on the three-dimensional location information of the trainee.

23. A system for training recognition ability, comprising:
an apparatus for training recognition ability comprising an instruction generating unit for generating a series of robot instruction information to instruct a behavior of a robot or a series of trainee instruction information to instruct a behavior of a trainee, a sensor unit for collecting three-dimensional location information of the trainee who imitates the behavior of the robot according to the robot instruction information or acts according to the trainee instruction information, and sensor information comprising color information or mobile device usage information of the trainee, a trainee behavior information generating unit for generating trainee behavior information based on the sensor information collected by the sensor unit, and a recognition ability determining unit for calculating recognition ability of the trainee based on the trainee behavior information along with the robot instruction information or the trainee instruction information;
a robot communicating with the apparatus for training recognition ability to operate according to the series of robot instruction information; and
a mobile device used by the trainee and to which information is input according to the trainee instruction information.
